# EUROPEAN PATENT APPLICATION

(11) **EP 1 078 922 A1**
(43) Date of publication of application: **28.02.2001**
(21) Application number: 00904082.5
(22) Date of filing: 22.02.2000
(51) Int. Cl.: C07D 307/33, C07D 313/04, C07D 313/06

(54) **PROCESS FOR THE PREPARATION OF ESTERS OR LACTONES**

(30) Priority: 11.03.1999 JP 6472599
(71) Applicant: Daicel Chemical Industries, Ltd., Osaka 590-8501 (JP)
(72) Inventor: ISHII, Yasutaka, Osaka 569-1112 (JP); NAKANO, Tatsuya, Himeji-shi, Hyogo 670-0094 (JP)
(74) Representative: Grünecker, Kinkeldey, Stockmair & Schwanhäusser Anwaltssozietät
(86) International application number: JP0000988
(87) International publication number: WO0053593

(57) **Abstract**

A process for producing an ester or a lactone according to the invention allows a ketone represented by the following formula (1): 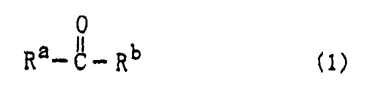 (wherein each of R^{a} and R^{b} is, identical to or different from each other, an organic group having a carbon atom at a bonding site with art adjacent carbonyl carbon atom, where R^{a} and R^{b} may be combined to form a ring with the adjacent carbonyl carbon atom) to react with hydrogen peroxide in the presence of a compound containing a metal element selected from Group 3, Group 13, Group 14, and Group 15 of the Periodic Table of Elements, to thereby yield a compound represented by the following formula (2) 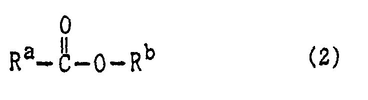 : (wherein each of R^{a} and R^{b} is, identical to or different from each other, an organic group having a carbon atom at a bonding site with an adjacent carbonyl carbon atom or oxygen atom, where R^{a} and R^{b} may be combined to form a ring with the adjacent carbonyl carbon atom and oxygen atom). The invention can produce a corresponding ester or lactone from a ketone in a good yield by use of hydrogen peroxide.

## Description

### Technical Field

The present invention relates to a process for producing an ester or a lactone. Specifically, the present invention relates to a process for producing a corresponding ester or lactone by oxidation of a ketone with hydrogen peroxide.

### Background Art

Esters or lactones are important compounds as pharmaceuticals, perfumes, dyestuffs, intermediates in organic synthesis and materials for polymeric resins.

As a process for obtaining an ester or lactone, a process is known in which a chain or cyclic ketone is allowed to react with an organic peracid such as perbenzoic acid, peracetic acid or trifluoroperacetic acid. In this process, an ester or a lactone is formed by a Baeyer-Villiger rearrangement (oxidation). However, such an organic peroxide is relatively expensive and produces, as a by-product, an equivalent amount of a carboxylic acid as a result of reaction. In contrast, the use of hydrogen peroxide instead of the organic peracid in the above reaction produces an ester or a lactone in a very low yield. Such hydrogen peroxide is available at low cost and by-produces water instead of a carboxylic acid.

### Disclosure of Invention

Accordingly, it is an object of the present invention to provide a process for obtaining a corresponding ester or lactone from a ketone in a good yield by use of hydrogen peroxide.

After intensive investigations to achieve the above object, the present inventors found that the use of a specific metallic compound as a catalyst can efficiently convert a ketone into an ester or a lactone by action of hydrogen peroxide. The present invention has been accomplished based on these findings.

Specifically, the present invention provides a process for producing an ester or a lactone. The process includes the step of allowing a ketone represented by the following formula (1): 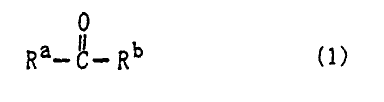 (wherein each of R^{a} and R^{b} is, identical to or different from each other, an organic group having a carbon atom at a bonding site with an adjacent carbonyl carbon atom, where R^{a} and R^{b} may be combined to form a ring with the adjacent carbonyl carbon atom)
to react with hydrogen peroxide in the presence of a compound containing a metal element selected from Group 3, Group 13, Group 14, and Group 15 of the Periodic Table of Elements, to thereby yield a compound represented by the following formula (2): 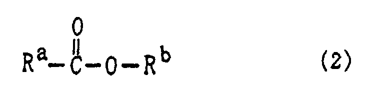 (wherein each of R^{a} and R^{b} is, identical to or different from each other, an organic group having a carbon atom at a bonding site with an adjacent carbonyl carbon atom or oxygen atom, where R^{a} and R^{b} may be combined to form a ring with the adjacent carbonyl carbon atom and oxygen atom).

In this process, an alicyclic ketone having 3 to 20 members may be allowed to react with hydrogen peroxide to thereby yield a corresponding lactone.

In this connection, the ketone for use as a reaction material may be referred to as "substrate" in the present description.

### Best Mode for Carrying Out the Invention

### [Substrate]

In the ketone represented by the formula (1), the "organic group having a carbon atom at a bonding site with an adjacent carbonyl carbon atom" represented by R^{a} and R^{b} includes hydrocarbon groups and heterocyclic groups. Such hydrocarbon groups include, but are not limited to, methyl, ethyl, propyl, isopropyl, butyl, isobutyl, s-butyl, t-butyl, pentyl, neopentyl, hexyl, octyl, decyl, dodecyl, pentadecyl, vinyl, allyl, 1-hexenyl, ethynyl, 1-butynyl, and other aliphatic hydrocarbon groups (alkyl groups, alkenyl groups, or alkynyl groups) each having about 1 to 20 carbon atoms (preferably 1 to 15 carbon atoms, arid more preferably 1 to 10 carbon atoms); cyclopropyl, cyclobutyl, cyclopentyl, cyclopentenyl, cyclohexyl, cyclohexenyl, cyclooctyl, cyclododecyl, and other alicyclic hydrocarbon groups (cycloalkyl groups or cycloalkenyl groups) each having about 3 to 20 members (preferably 3 to 15 members, and more preferably 5 to 8 members); and phenyl, naphthyl, and other aromatic hydrocarbon groups each having about 6 to 18 carbon atoms.

Heterocyclic rings corresponding to the heterocyclic groups include, but are not limited to, heterocyclic rings each containing an oxygen atom as a heteroatom (e.g., tetrahydrofuran, chroman, isochroman, furan, oxazole, isoxazole, 4-oxo-4H-pyran, benzofuran, isobenzofuran, and 4-oxo-4H-chromene), heterocyclic rings each having a sulfur atom as a heteroatom (e.g., thiophene, thiazole, isothiazole, thiadiazole, 4-oxo-4H-thiopyran, and benzothiophene), and heterocyclic rings each containing a nitrogen atom as a heteroatom (e.g., pyrrolidine, piperidine, piperazine, morpholine, indoline, pyrrole, pyrazole, imidazole, triazole, pyridine, pyridazine, pyrimidine, pyrazine, indole, quinoline, acridine, naphthyridine, quinazoline, and purine).

The substituents R^{a} and R^{b} may be combined to form a ring with an adjacent carbonyl carbon atom. Such rings include, but are not limited to, cyclopropane, cyclobutane, cyclopentane, cyclopentene, cyclohexane, cyclohexene, cyclooctane, cyclododecane, and other alicyclic hydrocarbon rings (cycloalkane rings or cycloalkene rings) each having about 3 to 20 members (preferably 3 to 15 members, and more preferably 3 to 12 members); norbornane ring, norbornene ring, adamantane ring, and other bridged cyclic hydrocarbon rings or bridged heterocyclic rings each having two to four rings; and tetrahydrofuran, chroman, isochroman, pyrrolidine, piperidine, and other non-aromatic heterocyclic rings each having about 5 to 8 members.

The organic groups and groups which may be formed by R^{a} and R^{b} together with the adjacent carbon atom may have a substituent. Such substituents include, but are not limited to, halogen atoms, hydroxyl group, mercapto group, oxo group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, sulfo group, alkyl groups (e.g., methyl, ethyl, t-butyl, and other C₁-C₄ alkyl groups), alkenyl groups (e.g., C₂-C₄ alkenyl groups), alkynyl groups (e.g., C₂-C₄ alkynyl groups), alicyclic hydrocarbon groups, aromatic hydrocarbon groups, and heterocyclic groups. An aromatic or non-aromatic ring (a hydrocarbon ring or heterocyclic ring) may be condensed to the aforementioned ring.

Typical examples of the ketones represented by the formula (1) include acetone, methyl ethyl ketone, methyl isopropyl ketone, methyl isobutyl ketone, methyl s-butyl ketone, methyl t-butyl ketone, methyl decyl ketone, diethyl ketone, ethyl isopropyl ketone, isopropyl butyl ketone, methyl vinyl ketone, methyl isopropenyl ketone, methyl cyclohexyl ketone, methyl phenyl ketone, methyl 2-methylphenyl ketone, methyl 2-pyridyl ketone, cyclohexyl phenyl ketone, and other chain ketones; cyclopropanone, cyclobutanone, cyclopentanone, cyclohexanone, 4-methylcyclohexanone, 4-chlorocyclohexanone, isophorone, cycloheptanone, cyclooctanone, cyclodecanone, cyclododecanone, cyclopentadecanone, 1,3-cyclohexanedione, 1,4-cyclohexanedione, 1,4-cyclooctanedione, 2,2-bis(4-oxocyclohexyl)propane, bis(4-oxocyclohexyl)methane, 4-(4-oxocyclohexyl)cyclohexanone, 2-adamantanone, and other cyclic ketones.

According to the invented production process, the use of a chain ketone as a substrate yields a corresponding ester, and the use of a cyclic ketone as a substrate yields a corresponding lactone having one more member than that of the substrate. The invented production process is particularly useful as a process for producing corresponding lactones (e.g., ε-caprolactones) by oxidation of alicyclic ketones (e.g., cyclohexanones and other cycloalkanones, and cyclic ketones corresponding to bridged cyclic hydrocarbons each having two to four rings) each having 3 to 20 members. The cyclic ketones may have a substituent such as alkyl groups.

### [Catalyst]

According to the present invention, compounds each containing a metal element selected from Group 3, Group 13, Group 14, and Group 15 of the Periodic Table of Elements are used as catalysts. Each of these compounds can be used alone or in combination. The term "compound(s)" as used in the present description also includes elementary metals.

Group 3 elements of the Periodic Table of Elements include rare earth elements [e.g., scandium, yttrium, lanthanum series elements (lanthanum, cerium, praseodymium, neodymium, promethium, samarium, europium, gadolinium, terbium, dysprosium, holmium, erbium, thulium, ytterbium, and lutetium)], and actinium series elements (e.g., actinium). Preferred Group 3 elements of the Periodic Table of Elements include scandium, samarium, and other rare earth elements.

Group 13 elements of the Periodic Table of Elements include aluminium, gallium, indium, and thallium. Group 14 elements of the Periodic Table of Elements include tin and lead. Group 15 elements of the Periodic Table of Elements include antimony and bismuth.

In a compound containing the element, the valence of the element is not specifically limited and is, for example, about 0 to 4, and preferably about 2 or 3.

Compounds containing the elements include elementary metals, hydroxides, oxides (including complex oxides), halides (fluorides, chlorides, bromides, and iodides), salts of oxoacids (e.g., nitrates, sulfates, phosphates, borates, and carbonates), oxoacids or salts thereof, isopolyacids or salts thereof, heteropolyacids or salts thereof each containing the element, and other inorganic compounds; salts of organic acids (e.g., prussiates; acetates, trichloroacetates, trifluoroacetates, propionates, naphthenates, stearates, maleates, tartrates, and other salts of carboxylic acids; methanesulfonates, trifluoromethanesulfonates, ethanesulfonates, benzenesulfonates, p-toluenesulfonates, and other sulfonates), complexes, and other organic compounds. Ligands for constituting the complexes include OH (hydroxo), alkoxy (e.g., methoxy, ethoxy, propoxy, and butoxy), acyl (e.g., acetyl and propionyl), alkoxycarbonyl (e.g., methoxycarbonyl and ethoxycarbonyl), acetylacetonato, cyclopentadienyl group, C₁-C₄ alkyl-substituted dicyclopentadienyl (e.g., pentamethyldicyclopentadienyl), C₁-C₄ alkyl (e.g., methyl and ethyl), halogen atoms (e.g., chlorine and bromine), CO, CN, oxygen atom, H₂O (aquo), phosphines (triphenylphosphine and other triarylphosphines), and other phosphorus compounds, NH₃ (ammine), NO, NO₂ (nitro), NO₃ (nitrato), ethylenediamine, diethylenetriamine, pyridine, phenanthroline, and other nitrogen-containing compounds, tetrahydrofuran and other oxygen-containing compounds.

Of these compounds, chlorides and other halides, sulfates and other salts of oxoacids, sulfonates and other salts of organic acids are preferred.

Typical examples of compounds of Group 3 elements of the Periodic Table of Elements include, by taking samarium compounds as example, samarium oxide, samarium iodide, samarium bromide, samarium chloride, samarium nitrate, samarium sulfate, samarium phosphate, samarium carbonate, and other inorganic compounds; samarium acetate, samarium trichloroacetate, samarium trifluoroacetate, samarium trifluoromethanesulfonate, acetylacetonatosamarium, and other organic compounds. In these compounds, the samarium usually has a valence of 2 or 3. Compounds of the other Group 3 elements of the Periodic Table of Elements than samarium include compounds corresponding to the samarium compounds.

Typical examples of compounds of Group 13 elements of the Periodic Table of Elements include, but are not limited to, aluminium oxide, aluminium iodide, aluminium bromide, aluminium chloride, aluminium fluoride, aluminium nitrate, aluminium sulfate, aluminium phosphate, and other inorganic aluminium compounds; aluminium acetate, aluminium trifluoroacetate, aluminium trifluoromethanesulfonate, and other organic aluminium compounds; indium oxide, indium iodide, indium bromide, indium chloride, indium fluoride, indium nitrate, indium sulfate, indium phosphate, and other inorganic indium compounds; and corresponding gallium or thallium compounds. The metal elements in these compounds usually have a valence of 3.

Typical examples of compounds of Group 14 elements of the Periodic Table of Elements include, but are not limited to, tin oxide, tin iodide, tin bromide, tin chloride, tin fluoride, tin nitrate, tin sulfate, and other inorganic tin compounds; tin acetate, tin trifluoroacetate, tin trifluoromethanesulfonate, acetylacetonatotin, and other organic tin compounds; lead oxide, lead iodide, lead bromide, lead chloride, lead fluoride, lead nitrate, lead sulfate, lead carbonate, and other inorganic lead compounds; lead acetate, and other organic lead compounds. The tin in the tin compounds usually has a valence of 2 or 3, and the lead in the lead compounds generally has a valence of 2 or 4.

Typical examples of compounds of Group 15 elements of the Periodic Table of Elements include, but are not limited to, antimony oxide, antimony iodide, antimony bromide, antimony chloride, antimony fluoride, antimony sulfate, and other trivalent or pentavalent antimony compounds; bismuth oxide, bismuth iodide, bismuth bromide, bismuth chloride, bismuth fluoride, bismuth nitrate, bismuth sulfate, and other trivalent bismuth compounds.

The catalyst may be supported on a carrier prior to use. As the carrier, activated carbon, silica, alumina, silica-alumina, zeolite, and other porous carriers are employed in many cases. The amount of the catalytic component (compound containing the element) to be supported on a carrier is, for example, about 0.1 to 50 parts by weight, and preferably about 0.5 to 30 parts by weight relative to 100 parts by weight of the carrier.

The proportion of the compound containing the element can be selected within a wide range and is, for example, about 0.00001 to 1 mole, preferably about 0.001 to 0.5 mole, and more preferably about 0.01 to 0.25 mole per 1 mole of the compound represented by the formula (1).

### [Hydrogen Peroxide]

The hydrogen peroxide for use in oxidation of the substrate is not specifically limited, but it is often used as an aqueous solution. For example, a commercially available about 35% by weight aqueous hydrogen peroxide solution can be employed as intact or can be appropriately diluted prior to use.

The amount of the hydrogen peroxide can be appropriately selected depending on the type of the substrate and generally is about 0.9 mole or more (e.g., about 0.9 to 10 moles), and more preferably about 1 to 5 moles per 1 mole of the substrate.

### [Reaction]

A reaction of the compound represented by the formula (1) with hydrogen peroxide is usually performed in the presence of a solvent. Such solvents include, but are not limited to, water; hexane, octane, and other aliphatic hydrocarbons; benzene, toluene, and other aromatic hydrocarbons; chloroform, dichloromethane, dichloroethane, carbon tetrachloride, chlorobenzene, trifluoromethylbenzene, and other halogenated hydrocarbons; diethyl ether, diisopropyl ether, dimethoxyethane, tetrahydrofuran, dioxane, and other ethers; acetic acid, propionic acid, and other organic acids; ethyl acetate, butyl acetate, and other esters; acetonitrile, propionitrile, benzonitrile, and other nitriles; methanol, ethanol, and other alcohols; dimethylformamide (DMF), dimethylacetamide, N-methylpyrrolidone, dimethyl sulfoxide, and other aprotic polar solvents; and mixtures of these solvents. Preferred solvents include, for example, water, hydrophilic solvents (e.g., nitriles, ketones, ethers, and aprotic polar solvents), and mixtures of water with hydrophilic solvents.

A reaction temperature is, for example, about -20°C to 150°C, preferably about 0°C to 100°C, and more preferably about 10°C to 60°C. A reaction time can be appropriately selected within a range from 10 minutes to 48 hours. The reaction can be performed in any of batch system, semi-batch system and continuous system.

After the completion of reaction, reaction products can be easily separated and purified by a conventional technique such as filtration, concentration, distillation, extraction, crystallization, recrystallization, column chromatography, and other separation and purification means, or a combination of these means.

According to the invented process, a corresponding ester or lactone can be obtained from a ketone in a good yield by use of hydrogen peroxide.

The present invention will now be illustrated in further detail with reference to several examples below, which are not intended to limit the scope of the invention. Incidentally, reaction products were analyzed by gas chromatography.

### EXAMPLE 1

A mixture of 2 mmol of cyclobutanone, a 35% by weight aqueous hydrogen peroxide solution (H₂O₂: 6 mmol), 0.3 mmol of indium chloride (InCl₃), and 5 ml of acetonitrile was stirred at 25°C for 6 hours. As a result, γ-butyrolactone was obtained in a yield of 73%.

### EXAMPLE 2

The procedure of Example 1 was repeated, except that 2 mmol of cyclohexanone was used instead of cyclobutanone, to thereby yield ε-caprolactone in a yield of 21%.

### EXAMPLE 3

The procedure of Example 1 was repeated, except that 2 mmol of 3-methylcyclohexanone was used instead of cyclobutanone, to thereby yield β-methyl-ε-caprolactone and δ-methyl-ε-caprolactone in total yield of 17% (approximately 1:1).

### EXAMPLE 4

The procedure of Example 1 was repeated, except that 2 mmol of 4-methylcyclohexanone was used instead of cyclobutanone, to thereby yield γ-methyl-ε-caprolactone in a yield of 19%.

### EXAMPLE 5

The procedure of Example 1 was repeated, except that 2 mmol of 4-t-butylcyclohexanone was used instead of cyclobutanone, to thereby yield γ-t-butyl-ε-caprolactone in a yield of 38%.

### EXAMPLE 6

The procedure of Example 1 was repeated, except that 2 mmol of 2-adamantanone was used instead of cyclobutanone, to thereby yield a lactone compound represented by the following formula in a yield of 38%.

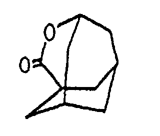

### EXAMPLE 7

A mixture of 2 mmol of cyclohexanone, a 35% by weight aqueous hydrogen peroxide solution (H₂O₂: 6 mmol), 0.3 mmol of samarium trifluoromethanesulfonate [Sm(OTf)₃], and 5 ml of acetonitrile was stirred at 25°C for 6 hours. As a result, ε-caprolactone was obtained in a yield of 15%.

### EXAMPLE 8

A mixture of 2 mmol of cyclohexanone, a 35% by weight aqueous hydrogen peroxide solution (H₂O₂: 6 mmol), 0.3 mmol of scandium trifluoromethanesulfonate [Sc(OTf)₃], and 5 ml of acetonitrile was stirred at 25°C for 6 hours. As a result, ε-caprolactone was obtained in a yield of 12%.

### EXAMPLE 9

A mixture of 2 mmol of cyclohexanone, a 35% by weight aqueous hydrogen peroxide solution (H₂O₂: 6 mmol), 0.3 mmol of aluminium chloride (AlCl₃), and 5 ml of acetonitrile was stirred at 25°C for 6 hours. As a result, ε-caprolactone was obtained in a yield of 12%.

### EXAMPLE 10

A mixture of 2 mmol of cyclohexanone, a 35% by weight aqueous hydrogen peroxide solution (H₂O₂: 2 mmol), 0.1 mmol of indium chloride (InCl₃), and 5ml of acetonitrile was stirred at 25°C for 6 hours. As a result, ε-caprolactone was obtained in a yield of 12%.

### EXAMPLE 11

A mixture of 2 mmol of cyclohexanone, a 35% by weight aqueous hydrogen peroxide solution (H₂O₂: 2 mmol), 0.1 mmol of bismuth chloride (BiCl₃), and 5 ml of acetonitrile was stirred at 25°C for 6 hours. As a result, ε-caprolactone was obtained in a yield of 8%.

### EXAMPLE 12

A mixture of 2 mmol of cyclohexanone, a 35% by weight aqueous hydrogen peroxide solution (H₂O₂: 2 mmol), 0.1 mmol of tin chloride (SnCl₂), and 5 ml of acetonitrile was stirred at 25°C for 6 hours. As a result, ε-caprolactone was obtained in a yield of 14%.

## Claims

1. A process for producing an ester or a lactone, comprising the step of allowing a ketone represented by the following formula (1): 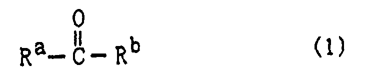 (wherein each of R^{a} and R^{b} is, identical to or different from each other, an organic group having a carbon atom at a bonding site with an adjacent carbonyl carbon atom, where R^{a} and R^{b} may be combined to form a ring with the adjacent carbonyl carbon atom)
to react with hydrogen peroxide in the presence of a compound containing a metal element selected from Group 3, Group 13, Group 14, and Group 15 of the Periodic Table of Elements, to thereby yield a compound represented by the following formula (2): 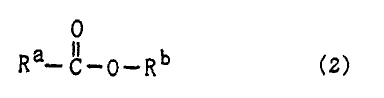 (wherein each of R^{a} and R^{b} is, identical to or different from each other, an organic group having a carbon atom at a bonding site with an adjacent carbonyl carbon atom or oxygen atom, where R^{a} and R^{b} may be combined to form a ring with the adjacent carbonyl carbon atom and oxygen atom).

2. A process for producing an ester or a lactone according to claim 1, wherein an alicyclic ketone having 3 to 20 members is allowed to react with hydrogen peroxide to yield a corresponding lactone.
